# EUROPEAN PATENT APPLICATION

(11) **EP 1 712 633 A1**
(43) Date of publication of application: **18.10.2006**
(21) Application number: 04819847.7
(22) Date of filing: 01.12.2004
(51) Int. Cl.: C12P 17/00, C12R 1/465, C12R 1/66, C12R 1/80

(54) **PROCESS FOR PRODUCING OPTICALLY ACTIVE TETRAHYDROTHIOPHENE DERIVATIVE AND METHOD OF CRYSTALLIZING OPTICALLY ACTIVE TETRAHYDROTHIOPHEN-3-OL**

(30) Priority: 02.12.2003 JP 2003403654; 26.01.2004 JP 2004017369
(71) Applicant: MERCIAN CORPORATION, Chuo-ku, Tokyo 104-8305 (JP)
(72) Inventor: NAGAI, Hazuki, Shizuoka, 4380078 (JP); KONUKI, Kaname, Shizuoka, 4380078 (JP); ITO, Shoji, Shizuoka, 4380078 (JP); SAMESHIMA, Tomohiro, Shizuoka, 4380077 (JP)
(74) Representative: Patentanwälte Ruff, Wilhelm, Beier, Dauster & Partner
(86) International application number: PCT/JP2004/017837
(87) International publication number: WO 2005/054491

(57) **Abstract**

A method for manufacturing (R)-tetrahydrothiophene-3-ol denoted by formula (II): 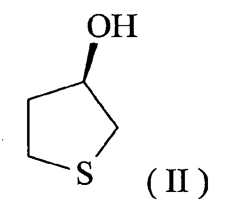 by bioconversion of tetrahydrothiophene-3-one denoted by formula (I): 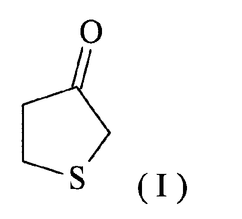 to (R)-tetrahydrothiophene-3-ol denoted by formula (II). It comprises the steps of: (A) incubating the tetrahydrothiophene-3-one denoted by formula (I) in the presence of a strain, or a preparation of a cultured cell thereof, belonging to *Penicillium, Aspergillus,* or *Streptomyces* that is capable of said bioconversion; and (B) collecting the (R)-tetrahydrothiophene-3-ol denoted by formula (II) from incubated solution. A method for crystallization of optically active tetrahydrothiophene-3-ol of improved optical purity, **characterized by** maintaining a solution comprising optically active tetrahydrothiophene-3-ol and organic solvent at equal to or lower than 1°C to cause optically active tetrahydrothiophene-3-ol to crystallize from said solution, or **characterized by** adding optically active tetrahydrothiophene-3-ol dropwise to organic solvent at a solution temperature of equal to or lower than 1°C to cause optically active tetrahydro thiophene-3-ol to crystallize.

## Description

### TECHNICAL FIELD

The present invention relates to a method for manufacturing (R)-tetrahydrothiophene-3-ol, that can be utilized as an intermediate in the synthesis of optically active pharmaceuticals, by bioconversion, and a method for crystallization of optically active tetrahydrothiophene-3-ol.

### BACKGROUND ART

(R)-tetrahydrothiophene-3-ol denoted by formula (II) is employed as an intermediate in the synthesis of penem-based antibiotics (see Japanese Unexamined Patent Publication (KOKAI) Showa No. 63-287781).

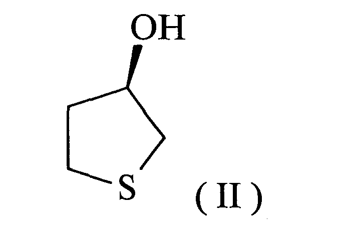

Known methods for manufacturing (R)-tetrahydrothiophene-3-ol include a method in which iridium catalysts are employed (see Japanese Unexamined Patent Publication (KOKAI) Heisei No. 04-139192), a method in which metal complex catalysts are employed (see Japanese Unexamined Patent Publication (KOKAI) Heisei No. 04-139140), a method in which synthesis is started from amino acids (see J. Org. Chem. 57, 4354 (1992)), a method in which synthesis is started from 2,3-dihydrothiophene (see J.Am.Chem.Soc. 108, 2049-2054 (1986)), and other methods based on chemical synthesis. However, these methods are problematic in that they require expensive reagents, afford low yields due to multiple reaction steps, and the like. They are not satisfactory as industrial production methods.

The method of esterifying racemic tetrahydrothiophene-3-ol and optically resolving the esters with an enzyme such as lipase is also known (see Biocatalysis 9, 61-69 (1994)). However, since this method is an optical resolution method using a racemic body as a starting material, the maximum theoretical yield of the targeted optically active product is 50 percent, rendering this method unsatisfactory in terms of the yield. Further, optical resolution methods using selective hydrolysis or esterification in which enzymes such as lipase are employed sometimes require a step of introducing a protective group onto the substrate or removal of protection or the like from the reaction product, in addition to enzyme reaction process. As a result, the steps are complex and such methods are not advantageous as industrial production methods.

Accordingly, it is a first object of the present invention to provide a novel method for manufacturing optically active tetrahydrothiophene-3-ol employing tetrahydrothiophene-3-one as a starting material by bioconversion with few of the problems (such as the formation of by-products in the form of optically active substances having a configuration reverse to that of the desired optically active product, the use of highly toxic compounds, and decreased selectivity) associated with the above-described stereoselective reduction methods and optical resolution methods.

In order to employ optically active tetrahydrothiophene-3-ol as an intermediate in the synthesis of pharmaceuticals, the optical purity thereof is desirably increased. A known method of increasing the optical purity of an optically active product, when the optically active product is an acid or basic substance, is to form diastereoisomer salts with the optically active acid or base and selectively crystallize one of the diastereoisomer salts based on differences in solubility. Further known methods include the use of chiral column chromatography and preferential crystallization employing highly optically pure seed crystal.

However, tetrahydrothiophene-3-ol is liquid at room temperature, so it is difficult to increase optical purity by common preferential crystallization methods. In addition, since it does not possess functional groups exhibiting marked acidity or basicity, such as carboxyl groups or amino groups, application of the method of forming diastereoisomer salts is also difficult. Furthermore, the method using chiral column chromatography is not suited to industrial production in terms of equipment or cost.

Accordingly, it is a second object of the present invention to provide a method for obtaining tetrahydrothiophene-3-ol of improved optical purity using optically active tetrahydrothiophene-3-ol as a starting material.

### DISCLOSURE OF THE INVENTION

To achieve the above-stated first object, the present inventors searched a large group of microorganisms for microorganisms capable of stereoselectively reducing the third position oxo group of tetrahydrothiophene-3-one denoted by formula (I) below for conversion to optically active (R)-tetrahydrothiophene-3-ol, discovering microorganisms having high selectivity; the first aspect of the present invention was devised on this basis.

The first aspect of the present invention is as follows.
[1] A method for manufacturing (R)-tetrahydrothiophene-3-ol denoted by formula (II): 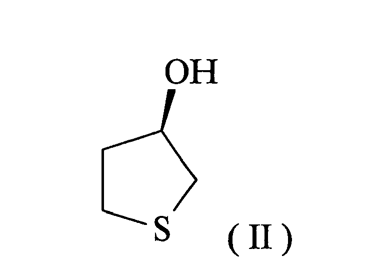 by bioconversion of tetrahydrothiophene-3-one denoted by formula (I): 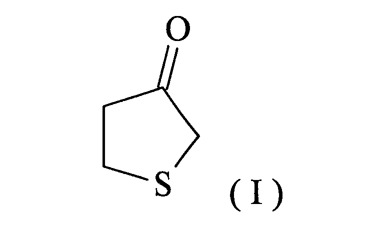 to (R)-tetrahydrothiophene-3-ol denoted by formula (II), comprising the steps of:
   (A) incubating the tetrahydrothiophene-3-one denoted by formula (I) in the presence of a strain, or a preparation of a cultured cell thereof, belonging to *Penicillium, Aspergillus,* or *Streptomyces* that is capable of said bioconversion; and
   (B) collecting the (R)-tetrahydrothiophene-3-ol denoted by formula (II) from incubated solution.
[2] The method of [1], wherein said strain capable of the bioconversion is a strain belonging to *Penicillium vinaceum, Aspergillus ochraceus,* or *Streptomyces michiganensis.*
[3] The method of [1] or [2], wherein said strain capable of the bioconversion is *Penicillium vinaceum* IAM7143 (Deposit Number: NITE BP-35), *Aspergillus ochraceus* ATCC18500 (deposit Number: NITE BP-41), or *Streptomyces michiganensis* NBRC12797 (Deposit Number: NITE BP-36).

Further, the present inventors have conducted extensive research and devised the second aspect of the present invention.

The second aspect of the present invention is as follows.
[4] A method for crystallization of optically active tetrahydrothiophene-3-ol of improved optical purity, characterized by maintaining a solution comprising optically active tetrahydrothiophene-3-ol and organic solvent at equal to or lower than 1°C to cause optically active tetrahydrothiophene-3-ol to crystallize from said solution.
[5] A method for crystallization of optically active tetrahydrothiophene-3-ol of improved optical purity, characterized by adding optically active tetrahydrothiophene-3-ol dropwise to organic solvent at a solution temperature of equal to or lower than 1°C to cause optically active tetrahydrothiophene-3-ol to crystallize.
[6] The method of [5], wherein said dropwise addition of optically active tetrahydrothiophene-3-ol is conducted with stirring said organic solvent.
[7] The method of any of [4] to [6], wherein the organic solvent is compatible with optically active tetrahydrothiophene-3-ol, and does not solidify at a temperature at which the crystallization is conducted.
[8] The method of any of [4] to [7], wherein the optically active tetrahydrothiophene-3-ol comprises excess amount of R-isomer.
[9] The method of any of [4] to [8], wherein said organic solvent is at least one solvent selected from the group consisting of hexane, heptane, ethyl acetate, butyl acetate, acetone, methyl ethyl ketone, ethanol, 2-propanol and toluene, or a mixed solvent thereof.
[10] The method of any of [4] to [9], wherein the crystallization temperature of said optically active tetrahydrothiophene-3-ol is equal to or lower than 1 °C.

### BEST MODE FOR CARRYING OUT THE INVENTION

### [First aspect]

The first aspect of the present invention is a method for manufacturing (R)-tetrahydrothiophene-3-ol denoted by formula (II): 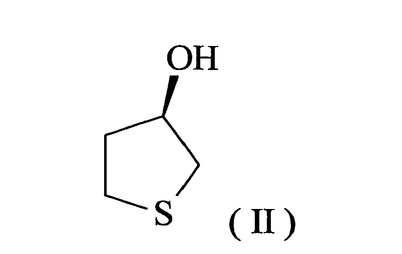 by bioconversion of tetrahydrothiophene-3-one denoted by formula (I): 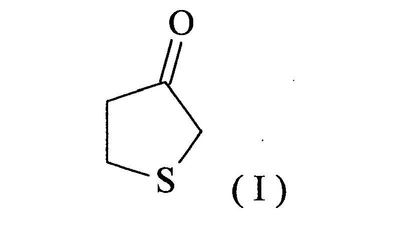 to (R)-tetrahydrothiophene-3-ol denoted by formula (II), comprising the steps of:
(A) incubating the tetrahydrothiophene-3-one denoted by formula (I) in the presence of a strain, or a preparation of a cultured cell thereof, belonging to *Penicillium, Aspergillus,* or *Streptomyces* that is capable of said bioconversion; and
(B) collecting the (R)-tetrahydrothiophene-3-ol denoted by formula (II) from incubated solution.

In the bioconversion of the present invention, cultured cells, or a preparation of the cultured cells, of a microorganism belonging to *Penicillium, Aspergillus,* or *Streptomyces* having the ability to convert the tetrahydrothiophene-3-one denoted by formula (I) above to the (R)-tetrahydrothiophene-3-ol denoted by formula (II) above can be employed regardless of species or strain. An enzyme catalyzing the above conversion reaction that has been isolated from these cells (referred to hereinafter as "tetrahydrothiophene-3-one reductase") may also be employed.

Preferred examples of such microorganisms are microorganisms belonging to *Penicillium vinaceum, Aspergillus ochraceus, Streptomyces michiganensis* and the like.

Specifically, particularly preferred examples among them are *Penicillium vinaceum* IAM7143, *Aspergillus ochraceus* ATCC18500, *Streptomyces michiganensis* NBRC12797, or the like.

Other microorganisms described above are preserved in preservation organizations of which names are given with strain names. The preservation organizations are as follows. IAM: Institute of Applied Microbiology, The University of Tokyo, ATCC: American Type Culture Collection, NBRC: Biotechnology Resource Center, Department of Biotechnology, Incorporated Administrative Agency, National Institute of Technology and Evaluation.

*Penicillium vinaceum* IAM7143 and *Streptomyces michiganensis* NBRC1279 have been deposited from November 16, 2004, and *Aspergillus ochraceus* ATCC18500 has been deposited from November 25, 2004 in Patent Microorganisms Depositary, Incorporate of Administrative Agency, National Institute of Technology and Evaluation (2-5-8 Kazusakamatari Kisarazu-shi, Chiba-Ken, Japan) respectively as NITE BP-35, NITE BP-36, and NITE BP-41.

According to the present invention, a starting material (substrate) in the form of tetrahydrothiophene-3-one is incubated in the presence of the cultured cells of a microorganism having the above-stated properties, or a preparation of such cultured cells. This process can be conducted by adding the substrate to the culture solution in the course of culturing the above-described microorganism, or following culturing. In some cases, the above cultured cells of the microorganism can be collected and employed as is or after being subjected to a pretreatment such as freeze-drying, spray drying, treatment with organic solvent (such as acetone), breaking treatment, or the like; or the tetrahydrothiophene-3-one reductase can be subjected to crude purification or purification isolation and then suspended in buffer solution, the substrate added, and the mixture incubated to conduct the reaction.

The substrate can be added to the culture solution either before culturing or after a prescribed time has elapsed following the start of culturing. Further, primarily for the purpose of promoting dissolution of the substrate and the like, it is possible to simultaneously add methanol, ethanol, methyl ethyl ketone, acetone, and the like; the present invention is not limited thereto. The cells can be produced by inoculating the above-described microorganism onto nutrient-containing medium and aerobically culturing the microorganism. Culturing of the microorganism to obtain a preparation of such cultured cells and culturing of the microorganism with the substrate added can in principle be conducted according to the general methods for culturing microorganisms. Normally, it is desirably conducted under aerobic conditions, such as shaking culturing, ventilated culturing with stirring or the like by liquid culture.

The medium employed in culturing need only permit the proliferation of the microorganism; various synthetic media, semisynthetic media, natural media and the like can be employed. Glucose, maltose, xylose, fructose, sucrose, starch, dextrin, glycerin, mannitol, oatmeal, and the like can be employed either singly or in combination as carbon sources in the culture composition.

Organic nitrogen sources such as peptones, meat extract, soy flour, casein, amino acids, wheat germ extract, yeast extract, urea, ammonium citrate and ammonium fumarate, as well as inorganic nitrogen sources such as sodium nitrate, potassium nitrate, ammonium sulfate, ammonium chloride, ammonium hydrogenphosphate and ammonium dihydrogenphosphate can be employed singly or in combination as nitrogen sources. Additionally, for example, salts such as sodium chloride, potassium chloride, calcium carbonate, magnesium sulfate, sodium phosphate, potassium phosphate, and cobalt chloride, as well as vitamins can be added for use as needed. When marked foaming occurs in the culture, various known antifoaming agents may be suitably added to the medium.

Examples of suitable media are: F1 medium (potato starch 20 g/L, glucose 10 g/L, soy flour 20 g/L, potassium dihydrogenphosphate 1 g/L, magnesium sulfate heptahydrate 0.5 g/L) and C medium (potato starch 20 g/L, glucose 20 g/L, soy flour 20 g/L, yeast extract 5 g/L, sodium chloride 2.5 g/L, calcium carbonate 3.2 g/L, metallic ion mixed solution 2 mL/L (composition of metallic ion mixed solution: copper sulfate pentahydrate 0.25 g/L, zinc sulfate heptahydrate 0.25 g/L, manganese chloride tetrahydrate 0.25 g/L)).

Culture conditions can be suitably selected top to the extent that microorganisms grow well. Culturing is normally conducted at pH 5.0 to 10.0, 20 to 30°C, preferably pH 6.5 to 8.0, 25 to 28°C, for 1 to 3 days, preferably about 3 days. The various culture conditions described above can be suitably varied based on the type and characteristics of microorganisms employed, external conditions, and the like, to select optimal conditions.

A preparation of cultured cell is prepared, after culturing has been completed, by suspending in a suitable solution either cells separated by centrifugal separation or filtration, or cells pretreated by freeze-drying, spray drying, processing with an organic solvent, breaking treatment, or the like. Solutions that are suitable for use in suspending of cells include the above-listed media, tris-acetic acid, tris-hydrochloric acid, sodium acetate, sodium citrate, sodium phosphate, potassium phosphate, and other buffer solutions, which may be employed singly or in combination. The pH of the buffer solution is preferably 5.0 to 9.0, more preferably 7.0 to 8.5. When employing the cultured cells as is, it is effective to add an energy source such as glucose and glycerol, and when employing a product of cultured cells, it is effective to add NAD(P)H or a coenzyme regenerating system. One example of a coenzyme regenerating system is the combination of glucose, glucose dehydrogenase, and NAD(P)⁺.

Tetrahydrothiophene-3-one serving as substrate can be added as is in liquid form or be diluted in a water-soluble organic solvent such as methanol, ethanol, acetone, dimethylformamide, or dimethylsulfoxide to the culture solution or cell suspension. The quantity added, for example, can be 0.1 to 100 g, preferably 1 to 20 g, per liter of culture solution when employing a culture solution. The substrate may be added all at once, but when the quantity being added is relatively large, the substrate can be added in several batches or continuously. Following addition of the substrate, operations such as shaking or aerated agitation can be conducted for 1 to 3 days, preferably 1 day, to promote the reaction and convert the substrate in the form of the tetrahydrothiophene-3-one denoted by formula (I) into the targeted (R)-tetrahydrothiophene-3-ol denoted by formula (II).

The targeted (R)-tetrahydrothiophene-3-ol thus produced can be separated from the reaction mixture by selecting and combining one or more of the various known purification methods. For example, adsorption onto and elution from hydrophobic adsorption resin; solvent extraction with ethyl acetate, n-butanol, or the like; column chromatography with silica gel or the like; thin-layer chromatography; high-performance liquid chromatography; distillation; and the like, may be employed singly or in suitable combination to separate and purify the product.

### [Second aspect]

The second aspect of the present invention is comprised of two modes:
a method for crystallization of optically active tetrahydrothiophene-3-ol of improved optical purity, characterized by maintaining a solution comprising optically active tetrahydrothiophene-3-ol and organic solvent at equal to or lower than 1°C to cause optically active tetrahydrothiophene-3-ol to crystallize from said solution (referred to as "crystallization method I", hereinafter); and
a method for crystallization of optically active tetrahydrothiophene-3-ol of improved optical purity, characterized by adding optically active tetrahydrothiophene-3-ol dropwise to organic solvent at a solution temperature of equal to or lower than 1°C to cause optically active tetrahydrothiophene-3-ol to crystallize (referred to as "crystallization method II", hereinafter).

In crystallization method I, crystallization of optically active tetrahydrothiophene-3-ol is conducted as follows. The starting material, optically active tetrahydrothiophene-3-ol is mixed with organic solvent to prepare a mixed solution of the optically active tetrahydrothiophene-3-ol and the organic solvent. The mixed solution containing the optically active tetrahydrothiophene-3-ol obtained is maintained at equal to or lower than 1°C, preferably at -3 to -18°C, to cause optically active tetrahydrothiophene-3-ol of improved optical purity to crystallize.

In crystallization method II, the starting material, optically active tetrahydrothiophene-3-ol is added dropwise to organic solvent at a solution temperature of equal to or lower than 1°C, preferably -10 to -18°C, desirably while stirring the organic solvent, to cause tetrahydrothiophene-3-ol of improved optical purity to crystallize. Neither the rate of dropwise addition of the starting material nor the quantity added is specifically limited; these may be suitably set.

In crystallization method I, while maintaining mixed solution of optically active tetrahydrothiophene-3-ol and organic solvent at equal to or lower than 1°C, the solution is desirably stirred.
In crystallization method II, it is desirable to add the optically active tetrahydrothiophene-3-ol dropwise while stirring the organic solvent. It is further desirable to continue stirring after the dropwise addition. In crystallization method II as in crystallization method I, the solution following dropwise addition of the starting material is preferably maintained at equal to or lower than 1°C, more preferably -10 to -18°C.

So long as it is not racemic, the optically active tetrahydrothiophene-3-ol serving as the starting material in both crystallization methods I and II can be of any optical purity, but is desirably 75 percent e.e. or greater. It is possible to employ optically active tetrahydrothiophene-3-ol of such optical purity regardless of the manufacturing method employed to produce it. For example, starting material produced by the above-described chemical synthesis, optical resolution methods employing enzymes, bioconversion methods, and more specifically, the method of the first aspect of the present invention, can be employed. The optically active tetrahydrothiophene-3-ol employed in crystallization methods I and II may be either R-isomer or S-isomer.

The organic solvent employed in crystallization methods I and II need only be compatible with optically active tetrahydrothiophene-3-ol at room temperature and not solidify at a temperature at which the crystallization is conducted. Examples of preferred organic solvents are hexane, heptane, toluene, acetone, methyl ethyl ketone, ethyl acetate, butyl acetate, ethanol, and 2-propanol. Normally, these solvents may be employed singly or in combinations of two or more. The use of a mixed solvent of hexane and ethyl acetate or a mixed solvent of hexane and acetone is preferred. For example, the mixture of hexane and acetone is desirably in a ratio of about 2:1 to 5:1.

The crystal of optically active tetrahydrothiophene-3-ol that has been crystallized in this manner can be separated and recovered at a temperature of equal to or lower than 1°C by the usual separation and recovery means, such as filtration and centrifugation.

### EXAMPLES

The present invention will be described in greater detail below based on specific examples. However, the present invention is not limited to these examples.

### Example 1

A 25 mL quantity of F1 medium (potato starch 20 g/L, glucose 10 g/L, soy flour 20 g/L, potassium dihydrogenphosphate 1 g/L, magnesium sulfate heptahydrate 0.5 g/L) was poured into a 250 mL conical flask and sterilized with high-pressure steam for 20 minutes at 121°C. This mixture was inoculated with *Aspergillus ochraceus* ATCC18500, and shaking culturing was conducted for 72 hours at 25°C. A 30 mg quantity of tetrahydrothiophene-3-one was added to the culture solution obtained, and the mixture was shaken for 24 hours at 25°C.

The reaction solution obtained was extracted with ethyl acetate (15 mL x 3). The organic layers were combined and dried over sodium sulfate and then concentrated after filtration. The residue was separated and purified by preparative TLC (hexane/ethyl acetate = 1/1) to give 12 mg of the target product (a yield of 39 percent) with an optical purity of 81 percent e.e. (R).; [α]_{D}= + 8.8(c=0.5, CHCl₃); ¹H-NMR(CDCl₃) δ(ppm): 1.75(m, 2H), 2.15(m, 1H), 2.82-3.17(m, 4H), 4.5(m, 1H).

In the present invention, optical purity was determined by high-performance liquid column chromatography (mobile phase: hexane/isopropanol = 96/4, flow rate: 1 mL/minute, temperature: 30°C, detection: 210 nm) equipped with a chiral column (Chiralpak AS-H (φ 0.46 x 25 cm) made by Daicel Chemical Industries). The absolute configuration was determined by comparison with the optically rotation given in J. Am. Chem. Soc. 108, 2049 (1986).

### Example 2

With the exceptions that *Penicillium vinaceum* IAM7143 was employed and a 50 mg quantity of substrate was added, the conversion reaction was conducted in the same manner as in Example 1. This yielded 11 mg of target product (a yield of 22 percent) with an optical purity of 91 percent e.e. (R).

### Example 3

With the exception that *Streptomyces michiganensis* NBRC12797 was employed, the conversion reaction was conducted in the same manner as in Example 2. This yielded 32 mg of target product (a yield of 79 percent) with an optical purity of 88 percent e.e. (R).

### Example 4

F1 medium (potato starch 20 g/L, glucose 10 g/L, soy flour 20 g/L, potassium dihydrogenphosphate 1 g/L, magnesium sulfate heptahydrate 0.5 g/L) that had been sterilized with high-pressure steam for 20 minutes at 121°C was poured in 1.5 L quantities into each of three mini-jars of three-liter capacity. The media were inoculated with *Penicillium vinaceum* IAM7143, and shaking culturing was conducted for 72 hours at 25°C. A 4 mL quantity of tetrahydrothiophene-3-one was added to each of the culture solutions obtained, and the mixtures were shaken for 24 hours at 20 to 23°C.

The reaction solution obtained from one of the three-liter mini-jars was extracted with ethyl acetate (total 1.75 L). The same extract operation was conducted for the remaining two mini-jars. The organic layers were combined, dried over sodium sulfate, and then concentrated after filtration. The residue was purified by distillation. This yielded 8.0 g (a yield of 55 percent) of (R)-tetrahydrothiophene-3-ol with an optical purity of 87 percent e.e.

### Example 5

Hexane (6.8 mL) and acetone (2.8 mL) were added to 8 g of the (R)-tetrahydrothiophene-3-ol with an optical purity of 87 percent e.e. from Example 4. The mixture was stirred overnight at-15°C. Stirring was continued overnight in this state. The white crystal that precipitated out was collected by filtration with a Kiriyama funnel (φ 40 mm, No. 4 filter paper). The crystal obtained by filtration gradually became liquid (R)-tetrahydrothiophene-3-ol.

5.7g (a yield of 71 percent); optical purity of 95%e.e.; [α]D=+11.6(c=0.1, CHCl₃); ¹H-NMR (CDCl₃) δ(ppm): 1.75(m,2H), 2.15(m,1H), 2.82-3.17(m, 4H), 4.5(m,1H).

### Example 6

(R)-tetrahydrothiophene-3-ol (50 g) with an optical purity of 91.8 percent e.e. was added to a mixed solution of acetone (217 mL) and heptane (43 mL). This mixture was gradually cooled with stirring to a final temperature of -15°C. Stirring was continued overnight in this state. The white crystal that precipitated out were collected by filtration with a Kiriyama funnel (φ 40 mm, No. 4 filter paper). After thoroughly removing the solvent from the crystal that had been obtained by filtration, the crystal was heated to obtain 31.6 g (a yield of 63 percent) of liquid (R)-tetrahydrothiophene-3-ol with an optical purity of 96 percent e.e.

### Example 7

Acetone (23.6 mL) and hexane (100 mL) were charged to a 300mL container, and the mixture was cooled to -18°C. (R)-tetrahydrothiophene-3-ol (122.32 g) with an optical purity of 91.8 percent e.e. was added dropwise with stirring. The container employed for the dropwise addition was washed with acetone (11.8 mL). Following overnight stirring, crystal was collected by filtration with a Kiriyama funnel (φ 60 mm, No. 5B filter paper). After thoroughly removing the solvent from the crystal that had been obtained by filtration, the crystal was heated to obtain 86.0 g (a yield of 70 percent) of liquid (R)-tetrahydrothiophene-3-ol with an optical purity of 97 percent e.e.

### Example 8

Methyl ethyl ketone (23.5 mL) and hexane (100 mL) were charged to a 300mL container, and the mixture was cooled to -18°C. (R)-tetrahydrothiophene-3-o1 (128.03 g) with an optical purity of 91.8 percent e.e. was added dropwise with stirring. The container employed for the dropwise addition was washed with acetone (11.8 mL). Following overnight stirring, crystal was collected by filtration with a Kiriyama funnel (φ 60 mm, No. 5B filter paper). After thoroughly removing the solvent from the crystal that had been obtained by filtration, the crystal was heated to obtain 83.9 g (a yield of 66 percent) of liquid (R)-tetrahydrothiophene-3-ol with an optical purity of 96 percent e.e.

### Example 9

With the exceptions that (R)-tetrahydrothiophene-3-ol (1.37 g) with an optical purity of 92 percent e.e., hexane (1.2 mL) and acetone (0.48 mL) were employed as well as crystallization was conducted at 1°C, the same processing was conducted as in Example 5. This yielded (R)-tetrahydrothiophene-3-ol of 95 percent e.e. at a yield of 65 percent.

### INDUSTRIAL APPLICABILITY

According to the present invention, (R)-tetrahydrothiophene-3-ol with high optical purity, that is useful as an intermediate in the synthesis of pharmaceuticals can be obtained.

## Claims

1. A method for manufacturing (R)-tetrahydrothiophene-3-ol denoted by formula (II): 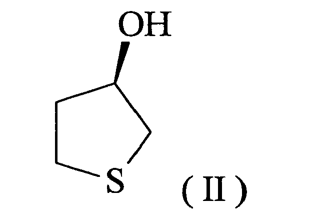 by bioconversion of tetrahydrothiophene-3-one denoted by formula (I): 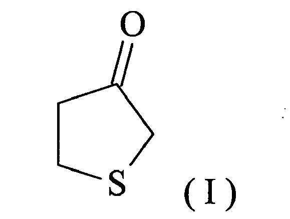 to (R)-tetrahydrothiophene-3-ol denoted by formula (II), comprising the steps of:
(A) incubating the tetrahydrothiophene-3-one denoted by formula (I) in the presence of a strain, or a preparation of a cultured cell thereof, belonging to *Penicillium, Aspergillus,* or *Streptomyces* that is capable of said bioconversion; and
(B) collecting the (R)-tetrahydrothiophene-3-ol denoted by formula (II) from incubated solution.

2. The method according to claim 1, wherein said strain capable of the bioconversion is a strain belonging to *Penicillium vinaceum, Aspergillus ochraceus,* or *Streptomyces michiganensis.*

3. The method according to claim 1 or 2, wherein said strain capable of the bioconversion is *Penicillium vinaceum* IAM7143 (Deposit Number: NITE BP-35), *Aspergillus ochraceus* ATCC18500 (deposit Number: NITE BP-41), or *Streptomyces michiganensis* NBRC12797 (Deposit Number: NITE BP-36).

4. A method for crystallization of optically active tetrahydrothiophene-3-ol of improved optical purity, **characterized by** maintaining a solution comprising optically active tetrahydrothiophene-3-ol and organic solvent at equal to or lower than 1°C to cause optically active tetrahydrothiophene-3-ol to crystallize from said solution.

5. A method for crystallization of optically active tetrahydrothiophene-3-ol of improved optical purity, **characterized by** adding optically active tetrahydrothiophene-3-ol dropwise to organic solvent at a solution temperature of equal to or lower than 1°C to cause optically active tetrahydrothiophene-3-ol to crystallize.

6. The method according to claim 5, wherein said dropwise addition of optically active tetrahydrothiophene-3-ol is conducted with stirring said organic solvent.

7. The method according to any of claims 4 to 6, wherein the organic solvent is compatible with optically active tetrahydrothiophene-3-ol, and does not solidify at a temperature at which the crystallization is conducted.

8. The method according to any of claims 4 to 7, wherein the optically active tetrahydrothiophene-3-ol comprises excess amount of R-isomer.

9. The method according to any of claims 4 to 8, wherein said organic solvent is at least one solvent selected from the group consisting of hexane, heptane, ethyl acetate, butyl acetate, acetone, methyl ethyl ketone, ethanol, 2-propanol and toluene, or a mixed solvent thereof.

10. The method according to any of claims 4 to 9, wherein the crystallization temperature of said optically active tetrahydrothiophene-3-ol is equal to or lower than 1 °C.
